# EUROPÄISCHE PATENTSCHRIFT

(11) **EP 0 530 934 B1**
(45) Veröffentlichungstag und Bekanntmachung des Hinweises auf die Patenterteilung: **08.11.1995**
(21) Anmeldenummer: 92250236.4
(22) Anmeldetag: 31.08.1992
(51) Int. Cl.: B07C 5/34, B29B 17/00, B07B 15/00, G01N 21/71, G01N 33/44

(54) **Verfahren zur Gewinnung sortenreiner Kunststofffraktionen**
Method for recovering pure fractions of plastics
Procédé pour extraire des fractions pures de maitières plastiques

(30) Priorität: 04.09.1991 DE 4129754
(43) Veröffentlichungstag der Anmeldung: 10.03.1993
(73) Patentinhaber: MAB-LENTJES ENERGIE- UND UMWELTTECHNIK GmbH, D-40880 Ratingen (DE)
(72) Erfinder: Dolle, Lothar, W-4690 Herne 1 (DE); Kirchgaesser, Peter, W-5000 Köln 60 (DE); Vollmer, Friedel, W-4690 Herne 2 (DE)
(74) Vertreter: Meissner, Peter E., Dipl.-Ing.

(56) Entgegenhaltungen:
- EP-A- 0 497 397
- EP-B- 0 293 983
- WO-A-92/07332
- DE-A- 2 552 481
- DE-A- 4 004 627
- US-A- 4 728 045
- US-A- 4 844 351
- US-A- 4 929 342

## Beschreibung

Die Erfindung betrifft ein Verfahren zur rechnergesteuerten Gewinnung sortenreiner Kunststofffraktionen aus zu einem großen Teil aus Kunststoffteilen, insbesondere aus Verpackungsmaterialien bestehenden Gemengen sowie eine Anlage zur Durchführung dieses Verfahrens.

Bei den Bemühungen um eine umweltverträgliche Entsorgung von Abfällen wird den Kunststoffen zunehmende Aufmerksamkeit geschenkt. Die zur Vermeidung einer einfachen Deponierung bisher übliche Verbrennung wird wegen des Risikos der Entstehung gasförmiger Schadstoffe, die eine entsprechend aufwendige Rauchgasreinigung erforderlich machen, häufig nicht mehr gewünscht. Die Errichtung von Verbrennungsanlagen stößt zunehmend auf Akzeptanzprobleme in der Bevölkerung. Statt dessen wird beabsichtigt, die in den Abfällen enthaltenen Wertstoffe auszusondern und einer Wiederverwertung zuzuführen. Eine Wiederverwertung von Kunststoffen in hochwertigen Produkten setzt jedoch voraus, daß diese in sortenreiner Form zur Verfügung stehen. Bei Verpackungsmaterialien, die in Gewerbebetrieben anfallen, kann vielfach wegen der Große und Homogenität der Verpackungen eine entsprechend sortenreine Erfassung von vornherein gewährleistet werden.

Dagegen ist eine solche sortenreine Erfassung beispielsweise bei Haushaltsabfällen derzeitig praktisch nicht möglich, da es nicht nur eine Vielzahl stofflich verschiedener Kunststoffverpackungen gibt, sondern auch mit einer fehlerhaften Zuordnung beim manuellen Sortieren gerechnet werden muß. Hinzu kommt, daß einem Haushalt nicht zugemutet werden kann, eine größere Vielzahl von unterschiedlichen Erfassungsbehältern zur Sammlung sortenreiner Kunststoffmaterialien bereitzuhalten. Heutige Abfallerfassungssysteme gehen daher höchstens nur so weit, daß sie eine grobe Vorsortierung bei der Erfassung beispielsweise in metallische Abfälle, Papier und Pappe, Kunststoffe und kompostierbare Abfälle vorsehen. Da ohnehin Zuordnungsfehlern in erheblichem Umfang Rechnung getragen werden muß, sieht eine in Deutschland geplante Abfallerfassungsmethode die Bereitstellung von Sammelbehältern für die privaten Haushalte vor, in denen lediglich zwei oder drei Fraktionen getrennt voneinander erfaßt werden sollen, nämlich die Verpackungsmaterialien und die sonstigen Abfälle sowie eventuell zusätzlich auch die kompostierbaren Abfälle.

Um die aus einer Gemengelage völlig unterschiedlicher Materialien bestehenden Verpackungsabfälle wiederverwerten zu können, müssen diese in möglichst sortenreine Fraktionen aufgeteilt werden. Im Grundsatz kann dies durch eine manuelle Sortierung gewährleistet werden, insbesondere wenn eine Kennzeichnungspflicht für Kunststoffe besteht. Wegen des damit verbundenen personellen Aufwandes und der vergleichsweise geringen Durchsatzleistung bleibt eine solche Sortiermöglichkeit praktisch aber auf die Aussortierung größerer Teile beschränkt. Eine entsprechende Aussortierung auch mittlerer und kleinerer Teile ist wirtschaftlich kaum vertretbar. Hinzu kommt der Umstand, daß manuelle Sortierarbeitsplätze aus der Sicht der Humanisierung der Arbeit wenig wünschenswert sind.

Bei der manuellen Sortierung kommt insbesondere im Hinblick auf die große Zahl unterschiedlicher Kunststoffe hinzu, daß bei der Erkennung des Materialtyps und bei der anschließenden Zuordnung zur sortenreinen Erfassung eine hohe Fehlerquote zu erwarten ist, die die Verwertbarkeit der gewonnenen Fraktionen beeinträchtigen.

Aufgabe der Erfindung ist es daher, ein Verfahren und eine Vorrichtung zu dessen Durchführung anzugeben, das eine möglichst automatische Sortierung von Kunststoffabfällen, die auch außerhalb der Hausmüllerfassung (z.B. Material aus Shredderanlagen) angefallen sein können, bei gleichzeitig geringer Fehlerquote gewährleistet.

Gelöst wird diese Aufgabe hinsichtlich des Verfahrens mit den Merkmalen des Patentanspruchs 1; zweckmäßige Weiterbildungen dieses Verfahrens sind durch die Merkmale der Unteransprüche 2 bis 11 näher gekennzeichnet. Eine erfindungsgemäße Vorrichtung weist die Merkmale des Patentanspruchs 12 auf und ist durch die Merkmale der Unteransprüche 13 bis 18 in vorteilhafter Weise ausgestaltbar.

Die Erfindung geht einerseits davon aus, daß es bekannt ist, zur Gewinnung von Wertstoffen aus Müll diesen manuell zu sortieren. Andererseits ist es bekannt, Stoffe anhand einer Spektralanalyse zu identifizieren. Nicht bekannt ist es jedoch, Kunststoffe in einem kontinuierlichen Durchlauf mit hoher Durchsatzgeschwindigkeit in jedem einzelnen Stück einer Spektralanalyse, insbesondere einer Analyse auf der Basis des Spektralbereichs des ultravioletten, des sichtbaren und des infraroten Lichtes zu unterziehen und das Ergebnis zur gezielten maschinellen Ausschleusung der einzelnen Teile im Sinne einer sortenreinen Erfassung zu nutzen. Da die Analysevorgänge und Ausschleusvorgänge sehr schnell erfolgen müssen, um eine für den großtechnischen Einsatz ausreichende Durchsatzmenge zu gewährleisten, ist eine Rechnersteuerung dieses Teils des erfindungsgemäßen Verfahrens vorgesehen.

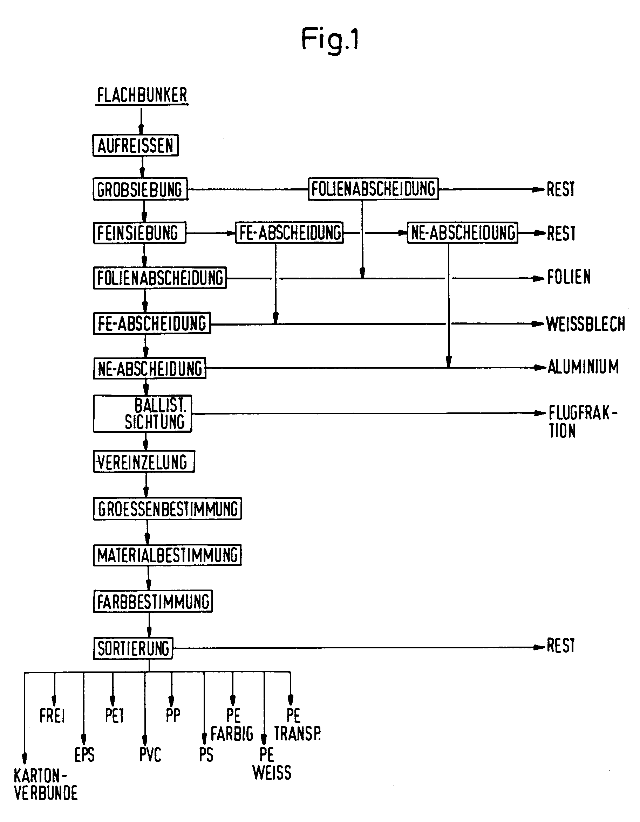

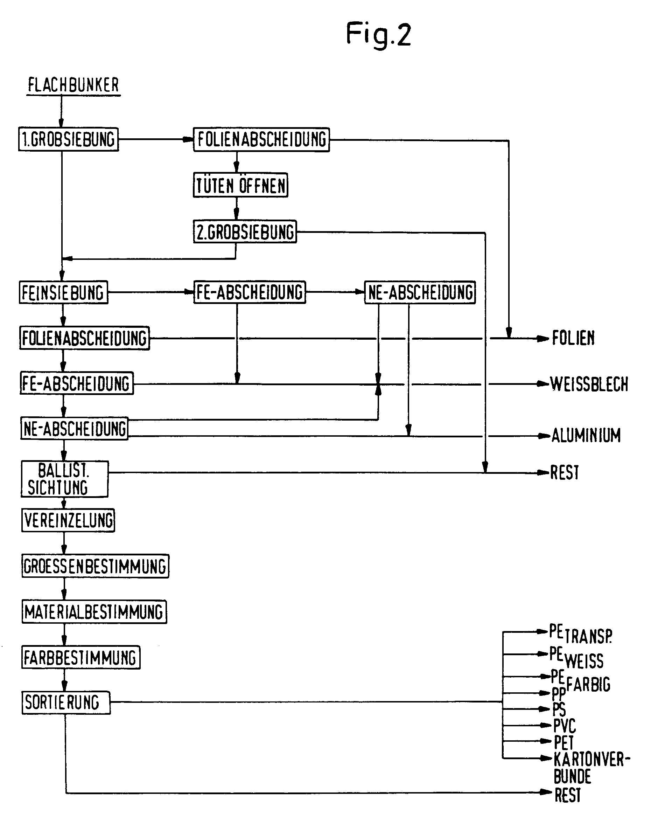

Im folgenden wird die Erfindung anhand der in den Figuren 1 und 2 jeweils in Form eines Verfahrensschemas wiedergegebenen Ausführungsbeispiele näher erläutert.

Aus einem Flachbunker werden die zu sortierenden Kunststoffabfälle entnommen. Kunststoffsäcke und -tüten werden zunächst geöffnet. Dies kann beispielsweise durch Aufreißen mittels feststehender oder bewegter Messer erfolgen. Es kann auch vorteilhaft sein, für das öffnen einen feinen Hochdruckwasserstrahl zu verwenden. Danach wird eine zweistufige Siebung (grob und fein) durchgeführt. Um besonders großstückige oder großflächige Teile (Grobfraktion) abzutrennen, die den weiteren Verfahrensgang stören könnten, weist das Grobsieb eine Maschenweite von 300 - 600 mm, vorzugsweise bis zu 500 mm, insbesondere bis zu 400 mm auf. Die Abtrennung der Grobfraktion könnte auch auf manuellem Wege durchgeführt werden oder wäre nicht notwendig, wenn bei der Abfallstoffsammlung von vornherein sichergestellt würde, daß die maximal zulässige Teilegröße nicht überschritten wird. Im Regelfall ist jedoch die beschriebene Art der Siebung vorzuziehen, zumal mit Fehlern bei der Bereitstellung der zu sortierenden Materialien zu rechnen ist. Die Grobfraktion mit den übergroßen Teilen wird einer Folienabscheidung unterzogen, und kann dann z.B. auf manuellem Wege einer Wiederverwertung zugeführt werden. Grundsätzlich wäre es auch möglich, das gesamte Material vor der Siebung durch eine Grobzerkleinerung gehen zu lassen. Das hätte aber den Nachteil, daß auch bereits ausreichend kleine Teile noch weiter zerkleinert würden und somit der nachfolgende Sortieraufwand wegen der Stückzahlerhöhung unnötig vergrößert würde. Außerdem würde die Fraktion des nicht verwertbaren Feinanteils unnötig vergrößert, also die Rückgewinnungsquote verschlechtert.

Der Unterlauf der Grobsiebung wird in einem nachgeschalteten Feinsieb weiter aufgeteilt in eine Mittel- und eine Feinfraktion. Die Feinfraktion aus der Feinsiebung wird durch einen Magnetabscheider und einen Nichteisenmetallabscheider von ferromagnetischen Stoffen (vorwiegend Weißblech) und Nichteisenmetallen befreit und aus dem Verfahren ausgeschleust.

Die gewonnene Mittelfraktion, die keine übergroßen Teile mehr enthält, wird ebenfalls wie die Grobfraktion durch eine Folienabscheidung, die beispielsweise auf pneumatischem Wege arbeitet, geführt. Die dabei abgeschiedenen Folien werden mit den übergroßen Folien aus der zuerstgenannten Folienabscheidung zusammengeführt und für eine Wiederverwertung bereitgestellt.

Danach wird auch die Mittelfraktion durch eine Magnetabscheidung (FE-Abscheidung) von ferromagnetischen Stoffen und durch eine NE-Abscheidung von anderen metallischen Stoffen (hauptsächlich Aluminium) befreit. Die NE-Abscheidung wird vorteilhaft in an sich bekannter Weise mit Hilfe der magnetischen Wirkung induzierter Wirbelströme vorgenommen. Entsprechende Anlagen sind beispielsweise bekannt aus der Veröffentlichung "Separationsanlagen für Nichteisenmetalle" (Aluminium, 65 (1989) 11, Seite 1125-1131). Die von Metallen befreite Mittelfraktion wird dann einer ballistischen Sichtung unterzogen, wobei eine vorwiegend aus Papier und restlichen Folien bestehende Flugfraktion abgeschieden wird. Die im Verfahren verbleibende Fraktion besteht fast vollständig aus Hartkunststoffen, die nachfolgend sortiert werden. Die in diesem Beispiel gewählte Reihenfolge der Verfahrensschritte Folienabscheidung, FE-Abscheidung und NE-Abscheidung ist nicht zwingend, sondern könnte auch geändert werden. Diese drei Verfahrensschritte sollten jedoch sinnvollerweise vor der ballistischen Sichtung ausgeführt werden.

Die Gewinnung sortenreiner Kunststofffraktionen beginnt mit einer nicht dargestellten Vereinzelung der Teile der verbleibenden Mittelfraktion, die strikt nacheinander einzeln in einem "kontinuierlichen Fluß" (z.B. auf einem Transportband) durch die weitere Anlage des erfindungsgemäßen Verfahrens geführt werden die von einem Rechner gesteuert wird. Die Vereinzelung kann beispielsweise in einfacher Weise durch zwei oder mehr hintereinander geschaltete Förderbänder mit jeweils höherer Geschwindigkeit realisiert werden. Nach der Vereinzelung wird zunächst die Größe des jeweils auf das Transportband gelangten Teils ermittelt (Größenbestimmung) und zeitkonform gespeichert. Die Größenbestimmung erfolgt auf optoelektronischem Wege, also berührungslos, beispielsweise mittels eines Kamerasystems oder eines Systems von Lichtschranken, die signaltechnisch mit dem Rechner verbunden sind.

Hierdurch wird der Rechner, der auch über die Geschwindigkeit des Transportbandes und deren Veränderungen laufend informiert ist, in die Lage versetzt, zu jedem beliebigen Zeitpunkt Ort und Lage der einzelnen Teile auf dem Transportband errechnen zu können. Dies ist wichtig für die nachfolgende Materialerkennung, die ebenfalls berührungslos durch eine Spektralanalyse erfolgt. Bekannt ist es, ein Spektrum dadurch zu erzeugen, daß mittels Elektroden ein Lichtbogen erzeugt wird, der eine geringe Menge des zu analysierenden Stoffs verdampft und ein elektromagnetische Wellen aussendendes Plasma erzeugt. Da der Elektrodenabstand zur Oberfläche des zu untersuchenden Teils vergleichsweise gering sein muß und im vorliegenden Fall die einzelnen zu identifizierenden Teile jeweils eine völlig unterschiedliche Geometrie aufweisen, wäre eine solche Art der Spektralanalyse wenig geeignet. Ebenfalls weniger für eine großtechnische Anwendung zweckmäßig wäre es, ein Spektrum z.B. in Form eines Infrarotspektrums zu erzeugen, in dem eine Durchstrahlung der einzelnen Teile mittels Infrarotlicht vorgenommen wird, da beispielsweise das Transportband hierbei hinderlich ist.

Demgegenüber ist es erfindungsgemäß zu bevorzugen, eine lasergestützte Emissionsspektroskopie anzuwenden, bei der mittels einer gepulsten Laserstrahlung eine kleine Materialprobe im Bereich der Oberfläche des zu untersuchenden Teils verdampft und in einen Plasmazustand überführt wird, so daß die von dem Plasma ausgehende Strahlung spektroskopisch untersucht werden kann.

Dies hat den großen Vorteil, daß der Abstand des Lasers von der Oberfläche des Teils relativ groß gehalten werden kann und der Strahlungskopf des Lasers nicht ständig auf einen neuen Abstand genau eingestellt werden muß.

Bekannt ist die lasergestützte Emissionsspektroskopie für die Identifizierung metallischer Werkstoffe, insbesondere von Stahl- und Aluminiumwerkstoffen. Für die Identifizierung von Kunststoffen wurde sie bisher nicht angewandt.

Erfindungsgemäß wird es besonders bevorzugt, die Identifizierung der Kunststoffart des jeweiligen Teils durch einen Vergleich von gemessenen Spektrallinien aus dem Bereich des ultravioletten, des sichtbaren und des infraroten Lichtes mit im Rechner gespeicherten Vergleichsspektren durchzuführen, die bestimmten Materialarten zugeordnet sind. Es hat sich überraschend herausgestellt, daß hierdurch eine sehr schnelle (Meßdauer unterhalb des Mikrosekundenbereichs), einfache und dennoch zuverlässige Materialidentifizierung möglich ist. Im einzelnen kann für die Identifizierung eine Vielzahl von Unterscheidungskriterien herangezogen werden, die sich aus den qualitativen und quantitativen Aussagen der Spektralanalyse ergeben. So ist beispielsweise für Kunststoffe wie PS, PVC, und PET das Zahlenverhältnis der Wasserstoffatome zu den Kohlenstoffatomen je Molekül bestimmend. Es beträgt 1 bzw. 1,5 bzw. 0,8. Für PVC ist außerdem die Spektrallinie des enthaltenen Chlor charakteristisch. Dagegen lassen sich PE und PP nicht durch das Verhältnis H : C unterscheiden, da es in beiden Fällen 2 beträgt. Dennoch ergeben sich aus der Spektralanalyse auch hierfür Unterscheidungsmerkmale. Ermittelt man nämlich die Fläche unter einer oder mehreren Linien von ausgewählten Bereichen im Spektrum und setzt diese ins Verhältnis zur Fläche unter einer oder mehreren Linien von ausgewählten Bereichen des Untergrundes, so ergeben sich für PE und PP jeweils signifikant unterschiedlich hohe Zahlenwerte. Diese liegen bei PP meistens um mindestens 50 % bzw. mindestens 30 % über denen des PE.

Da der Rechner, der den Materialidentifizierungsteil der Anlage steuert, über die örtliche Lage des zu untersuchenden Teils ständig informiert ist, kann er die Laserstrahlung so richten, bzw. zu einem geeigneten Zeitpunkt auslösen, daß sie sicher auf die Oberfläche dieses Teils auftrifft und die erforderliche Verdampfung einer kleinen Materialprobe bewirkt. Um die Meßgenauigkeit zu verbessern und von Zufallsergebnissen (z.B. geringfügige Materialinhomogenität, Oberflächenverschmutzungen, Farbaufdrucke usw.) unabhängig zu machen, sollten für jedes Teil mindestens zwei, besser noch drei oder mehr Messungen an verschiedenen Stellen der Oberfläche vorgenommen werden. Wenn jede Messung zu dem gleichen Ergebnis führt, kann eine völlig eindeutige Identifizierung durch den Rechner erfolgen. Ergeben sich stärkere Unterschiede, wird das jeweilige Teil als nicht identifizierbar vermerkt.

In vorteilhafter Weiterbildung der Erfindung weist das dargestellte Verfahrensschema in Transportrichtung hinter der Materialerkennung noch eine zusätzliche Farberkennung auf, die beispielsweise mittels einer Farbkamera oder besonders vorteilhaft anhand einer Spektralanalyse erfolgen kann.

Letzteres ist möglich, weil zur Einfärbung der Kunststoffe für bestimmte Farben üblicherweise jeweils ganz bestimmte Elemente verwendet werden, deren Spektren jeweils eindeutige Charakteristiken aufweisen. Zweckmäßigerweise wird die spektralanalytische Farberkennung zusammen mit der Materialarterkennung, also durch Auswertung desselben Spektrums durchgeführt. Das Farberkennungssystem ist in jedem Fall signaltechnisch mit dem Rechner verbunden, so daß das gemessene Ergebnis dem jeweiligen untersuchten Teil eindeutig zugeordnet werden kann. Auf diese Weise identifiziert der Rechner jedes Teil nicht nur nach Materialart, sondern im Bedarfsfall zusätzlich auch nach Farbe.

Mit diesen Informationen kann der Rechner eine Ausschleusvorrichtung ansteuern, so daß jedes einzelne Teil z.B. in einen ganz bestimmten Sammelbehälter oder auf ein Förderband mit eindeutiger Zuordnung zu einer bestimmten Materialart und ggf. darüberhinaus zu einer bestimmten Farbe gelenkt wird. Nicht identifizierbare Teile werden in einen gemeinsamen Sammelbehälter (Rest) befördert, von wo aus sie entweder von Hand weitersortiert oder einer Verbrennung oder auch einer Weiterverwendung mit besonders niedrigen Ansprüchen an die Materialgleichartigkeit (z.B. Verpressung zu Dämm-Material) zugeführt werden können. Im gezeigten Ausführungsbeispiel sind als Einzelfraktionen, die am Ende des Verfahrens als Wertstoffe gewonnen werden,, neben den Kartonverbundpackungen insgesamt neun Kunststofffraktionen dargestellt, wobei die Fraktion Polyäthylen (PE) in drei Einzelfraktionen für transparentes, weißes bzw. farbiges PE weiter aufgeteilt ist. Für die anderen Kunststoffarten wurde hier keine Farbaufteilung vorgesehen. Diese wäre ohne weiteres möglich und insbesondere dann sinnvoll, wenn der relative Mengenanteil dieser Kunststoffarten gegenüber dem zur Zeit klar dominierenden Anteil des Polyäthylen steigen würde.

Die Ausschleusvorrichtung wird zweckmäßigerweise als System von Pneumatikdüsen ausgebildet, die in Transportrichtung hintereinander seitlich am Transportband angeordnet sind und mit ihrer Blasrichtung quer zur Transportrichtung stehen. Auf der einer Düse gegenüberliegenden Seite ist jeweils ein Sammelbehälter oder ein Förderband für die Abförderung ausgeschleuster Teile angeordnet.

Wenn nun ein Teil gerade an dem Sammelbehälter oder dem Förderband vorbeiläuft, der bzw. das der Materialart und ggf. zusätzlich auch der Farbe dieses Teils eindeutig zugeordnet ist, dann wird durch den Rechner ein Absperrventil geöffnet, das Druckluft durch die jeweilige Düse strömen läßt und dadurch das Teil in die richtige Sammeleinrichtung für diesen Kunststoff bläst. Selbstverständlich könnte die Ausschleusvorrichtung auch anders ausgebildet sein und beispielsweise in das Förderband einschwenkbare Abweisklappen aufweisen, die im Sinne von Weichen eine gezielte Umlenkung einzelner Teile in eine bestimmte Richtung bewirken können. Das Pneumatikdüsensystem ist jedoch in der Regel wegen seiner Einfachheit, großen Störsicherheit und schnellen Ansprechbarkeit vorteilhafter.

Figur 2 zeigt ein Verfahrensschema der Erfindung das sich im wesentlichen nur im vorgeschalteten Aufbereitungsteil vor der ballistischen Sichtung unterscheidet. Die aus dem Flachbunker entnommenen Kunststoffabfälle werden zunächst in einem ersten Grobsieb von übergroßen Teilen (Siebüberlauf) befreit, wobei letztere zunächst einer pneumatischen Folienabscheidung unterzogen werden. Erst danach werden die in der Grobfraktion enthaltenen Tüten geöffnet und mit den übrigen Teilen der Grobfraktion einer zweiten Grobsiebung mit gegenüber dem ersten Grobsieb geringerer Maschenweite zugeführt. Der Siebüberlauf wird als Rest ausgeschleust, während der Siebunterlauf zusammen mit dem Siebunterlauf der ersten Grobsiebung einer Feinsiebung unterzogen wird.

Die dabei als Siebunterlauf abgetrennte Feinfraktion wird nach Aussonderung von Eisen und Nichteisenmetallen als nicht verwertbarer Rest ausgeschleust. Der Siebüberlauf der Feinsiebung stellt die verwertbare Mittelfraktion dar, die wie in Fig. 1 nach Abscheidung von Eisen- und Nichteisenmetallbestandteilen in die ballistische Sichtung gegeben und anschließend in der bereits beschriebenen Weise sortiert wird.

Eine Anlagenkonfiguration gemäß Fig. 2 hat gegenüber der Variante gemäß Fig. 1 den Vorteil, daß weniger und kürzere Transportbänder zur Verbindung der einzelnen Aggregate erforderlich sind. Dies ermöglicht ein kompakteres Layout der Anlage und verringert somit das umbaute Volumen; die Höhe des Investitionsaufwandes kann daher entsprechend gesenkt werden.

Mit der vorliegenden Erfindung ist es möglich, eine sortenreine Rückgewinnung von Kunststofffraktionen insbesondere aus Verpackungsabfällen in großtechnischem Maßstab bei hoher Rückgewinnungsquote auf rein maschinellem Wege durchzuführen, so daß eine fehlerbehaftete manuelle Sortierung, die eine aus humanitärer Sicht belastende Tätigkeit darstellt und sehr kostenaufwendig ist, unterbleiben kann. Durch die gewählte Analysemethode und die extrem schnelle Verarbeitung der erforderlichen Prozeßdaten in der Rechnersteuerung kann eine sehr hohe Sortiergeschwindigkeit und damit eine hohe Durchsatzleistung in einer erfindungsgemäßen Anlage gewährleistet werden.

## Patentansprüche

1. Verfahren zur rechnergesteuerten Gewinnung sortenreiner Kunststofffraktionen aus großenteils aus Kunststoffteilen, insbesondere aus Verpackungsmaterialien bestehenden Gemengen, gekennzeichnet durch die Kombination folgender Verfahrensschritte:
a) durch eine Grobsiebung werden mindestens die über 600 mm großen Teile des Gemenges als Grobfraktion aussortiert,
b) zur Abtrennung einer Feinfraktion von einer Mittelfraktion wird das verbleibende Gemenge aus Verfahrensstufe a) einer Feinsiebung mit einer Maschenweite von mindestens 15 mm bis maximal 50 mm unterzogen,
c) die Mittel- und die Grobfraktion werden einer Folienabscheidung unterzogen,
d) die Mittel- und die Feinfraktion werden zur Abtrennung ferromagnetischer Stoffe jeweils einer Magnetabscheidung unterzogen,
e) die Mittel- und die Feinfraktion werden jeweils einer Abscheidung von Nichteisenmetallen unterzogen,
f) die Mittelfraktion wird nach Durchführung der Verfahrensstufen a) bis e) einer ballistischen Sichtung unterzogen, in der als Flugfraktion insbesondere Papier und restliche Folien abgetrennt werden,
g) die Teile der verbleibenden Mittelfraktion aus Verfahrensstufe f) werden vereinzelt und strikt hintereinander durch die weiteren Verfahrensstufen transportiert,
h) die Größe der einzelnen Teile der vereinzelten Mittelfraktion wird jeweils von der Rechnersteuerung durch optoelektronische Messung erfaßt,
i) die Materialart jedes einzelnen Teils wird während des Transports durch eine Spektralanalyse bestimmt,
k) das Ergebnis der Spektralanalyse wird an die Rechnersteuerung gegeben und
l) entsprechend dem Ergebnis der Spektralanalyse wird von der Rechnersteuerung eine Ausschleusvorrichtung angesteuert, die das jeweilige Teil mit sortengleichen anderen Teilen in eine entsprechende Sammeleinrichtung befördert.

2. Verfahren nach Anspruch 1,
dadurch gekennzeichnet,
daß zur Durchführung der Spektralanalyse eine Materialprobe des jeweiligen Teils mittels eines von der Rechnersteuerung ausgelösten und auf das Teil gerichteten Laserstrahlimpulses verdampft und in ein Plasma umgewandelt wird.

3. Verfahren nach einem der Ansprüche 1 bis 2,
dadurch gekennzeichnet,
daß die Materialerkennung durch Vergleich der jeweils ermittelten Spektrallinien aus dem Bereich der ultravioletten, der sichtbaren und der infraroten Strahlung mit in der Rechnersteuerung gespeicherten Vergleichsspektren erfolgt, die einzelnen Kunststoffarten zugeordnet sind.

4. Verfahren nach einem der Ansprüche 1 bis 3,
dadurch gekennzeichnet,
daß die Materialbestimmung für jedes Teil durch mindestens 2, vorzugsweise 3 auf verschiedene Stellen des Teils gerichtete Probenahmen erfolgt.

5. Verfahren nach einem der Ansprüche 1 bis 4,
dadurch gekennzeichnet,
daß über die Spektralanalyse zur Erkennung der Materialart hinaus eine Farbbestimmung für das jeweilige Teil erfolgt, deren Ergebnis zur Erzielung einer zusätzlichen Sortierung der Teile nach Farben an die Rechnersteuerung gegeben wird.

6. Verfahren nach Anspruch 5,
dadurch gekennzeichnet,
daß die Farberkennung mit Hilfe einer Farbkamera erfolgt.

7. Verfahren nach Anspruch 5,
dadurch gekennzeichnet,
daß die Farberkennung durch Auswertung charakteristischer Ausschnitte des in der Spektralanalyse ermittelten Wellenspektrums erfolgt.

8. Verfahren nach einem der Ansprüche 1 bis 7,
dadurch gekennzeichnet,
daß die Erfassung der Teilegröße mittels Lichtschranken erfolgt.

9. Verfahren nach einem der Ansprüche 1 bis 7,
dadurch gekennzeichnet,
daß die Erfassung der Teilegröße mittels einer Kamera erfolgt.

10. Verfahren nach einem der Ansprüche 1 bis 9,
dadurch gekennzeichnet,
daß die Siebung zur Abtrennung der Feinfraktion mit einer Maschenweite von 25 - 40 mm durchgeführt wird.

11. Verfahren nach einem der Ansprüche 1 bis 10,
dadurch gekennzeichnet,
daß durch die Grobsiebung die maximale Teilegröße der Mittelfraktion auf 500 mm, vorzugsweise auf 400 mm beschränkt wird.

12. Anlage zur Durchführung des Verfahrens nach Anspruch 1, gekennzeichnet durch die Kombination folgender Merkmale:
- es sind mindestens ein Grobsieb mit einer Maschenweite von 300 - 600 mm und ein nachgeschaltetes Feinsieb mit einer Maschenweite von 15 - 50 mm zur Aufteilung des Gemenges in eine Grobfraktion, eine Mittelfraktion und eine Feinfraktion vorgesehen,
- hinter den beiden Sieben ist jeweils eine Vorrichtung zur Folienabscheidung aus der Mittel- und der Grobfraktion angeordnet,
- hinter den beiden Sieben ist jeweils eine Vorrichtung zur Aussonderung ferromagnetischer Stoffe aus der Mittel- und der Feinfraktion angeordnet,
- hinter den beiden Sieben ist außerdem jeweils eine Vorrichtung zur Aussonderung von Nichteisenmetallen aus der Mittel- und der Feinfraktion angeordnet,
- hinter dem NE-Metallabscheider und dem Magnetabscheider für die Mittelfraktion ist ein ballistischer Sichter zur Abtrennung von Papier und restlichen Kunststoffolien angeordnet,
- hinter dem Sichter ist eine Einrichtung zur vereinzelten Ausgabe der Kunststoffteile auf ein Transportband angeordnet, wobei die Vereinzelungseinrichtung eine strikte Hintereinanderanordnung der Kunststoffteile auf dem Transportband gewährleistet,
- in unmittelbarer Nähe des Transportbandes ist eine Einrichtung zur Größenerfassung der vereinzelten Kunststoffteile angeordnet,
- in Transportrichtung hinter der Größenerfassungseinrichtung ist eine Einrichtung zur berührungslosen Durchführung einer Spektralanalyse an den vereinzelten Kunststoffteilen angeordnet,
- in Transportrichtung hinter der Einrichtung zur Spektralanalyse ist eine mehrkanalige Ausschleusvorrichtung angeordnet, deren mehrere Ausschleuskanäle jeweils einem bestimmten Kunststoff zugeordnet sind und
- es ist eine Rechnersteuerung vorgesehen, die signaltechnisch mit der Transporteinrichtung, der Größenerfassungseinrichtung, der Einrichtung zur Spektralanalyse und der Ausschleusvorrichtung verbunden ist und artgleiche Kunststoffteile jeweils in gleicher Weise aus dem Transportband ausschleust zur Bildung sortenreiner Kunststofffraktionen.

13. Anlage nach Anspruch 12,
dadurch gekennzeichnet,
daß die Einrichtung zur Spektralanalyse ein Spektrometer für den Bereich der ultravioletten, sichtbaren und infraroten Strahlung ist, der die Erzeugung des Spektrums mittels einer gepulsten Laserstrahlung vornimmt.

14. Anlage nach einem der Ansprüche 12 bis 13,
dadurch gekennzeichnet,
daß die Einrichtung zur Größenbestimmung der Kunststoffteile als Lichtschrankensystem oder als Kamerasystem ausgebildet ist.

15. Anlage nach einem der Ansprüche 12 bis 14,
dadurch gekennzeichnet,
daß vor der Ausschleusvorrichtung eine mit der Rechnersteuerung signaltechnisch verbundene Einrichtung zur Farberkennung der Kunststoffteile angeordnet ist.

16. Anlage nach einem der Ansprüche 12 bis 15,
dadurch gekennzeichnet,
daß die Maschenweite des Feinsiebs 25 mm bis 40 mm beträgt.

17. Anlage nach einem der Ansprüche 12 bis 16,
dadurch gekennzeichnet,
daß die Maschenweite des Grobsiebs auf 500 mm, vorzugsweise auf 400 mm beschränkt ist.

18. Anlage nach einem der Ansprüche 12 bis 17,
dadurch gekennzeichnet,
daß die Ausschleusvorrichtung als System von in Transportrichtung hintereinander angeordneten Druckluftdüsen ausgebildet ist, deren Blasrichtung jeweils quer zur Transportrichtung steht und die jeweils separaten Sammeleinrichtungen zugeordnet sind.

## Claims

1. A method for the computer-controlled recovery of pure fractions of plastics materials from batches consisting largely of pieces of plastic, in particular of packaging materials, characterised by the combination of the following process steps:
a) at least the pieces of the batch which are over 600 mm are eliminated as coarse fraction by coarse screening,
b) in order to separate off a fine fraction from a medium fraction, the remaining batch from process stage a) is subjected to fine screening with a mesh width of at least 15 mm to at most 50 mm,
c) the medium and the coarse fraction are subjected to film separation,
d) the medium and the fine fraction are each subjected to magnetic separation in order to separate off ferromagnetic substances,
e) the medium and the fine fraction are each subjected to separation of non-ferrous metals,
f) the medium fraction is subjected to ballistic sorting once process stages a) to e) have been effected, in which in particular paper and other films are separated off as airborne fraction,
g) the pieces of the remaining medium fraction from process stage f) are separated and transported through the other process stages strictly one behind the other,
h) the size of the individual pieces of the separated medium fraction is detected in each case by the computer control means by optoelectronic measurement,
i) the type of material of each individual piece is determined during transport by spectral analysis,
k) the result of the spectral analysis is passed to the computer control means, and
l) corresponding to the result of the spectral analysis, a discharge device is triggered by the computer control means, which device conveys the respective piece with other pieces of the same type into a corresponding collecting means.

2. A method according to Claim 1, characterised in that in order to effect the spectral analysis a sample of material of the respective piece is vaporised by means of a laser beam pulse triggered by the computer control means and directed at the piece and is converted into a plasma.

3. A method according to one of Claims 1 to 2, characterised in that the recognition of material is effected by comparing the spectral lines determined in each case from the region of ultraviolet, visible and infrared radiation with comparison spectra which are allocated to individual types of plastics and are stored in the computer control means.

4. A method according to one of Claims 1 to 3, characterised in that the determination of material for each piece is effected by at least 2, preferably 3, sampling operations directed at different points on the piece.

5. A method according to one of Claims 1 to 4, characterised in that in addition to the spectral analysis for recognition of the type of material, colour determination for the respective piece is effected, the result of which is passed to the computer control means in order to achieve additional sorting of the pieces according to colour.

6. A method according to Claim 5, characterised in that the colour recognition is effected with the aid of a colour camera.

7. A method according to Claim 5, characterised in that the colour recognition is effected by evaluating characteristic sections of the wave spectrum determined in the spectral analysis.

8. A method according to one of Claims 1 to 7, characterised in that the detection of the piece size is effected by means of light barriers.

9. A method according to one of Claims 1 to 7, characterised in that the detection of the piece size is effected by means of a camera.

10. A method according to one of Claims 1 to 9, characterised in that the sifting for separating off the fine fraction is effected with a mesh width of 25 - 40 mm.

11. A method according to one of Claims 1 to 10, characterised in that the coarse screening restricts the maximum piece size of the medium fraction to 500 mm, preferably to 400 mm.

12. An installation for performing the method according to Claim 1, characterised by the combination of the following features:
- at least one coarse sieve having a mesh width of 300 to 600 mm and a subsequent fine sieve having a mesh width of 15 - 50 mm are provided for dividing up the batch into a coarse fraction, a medium fraction and a fine fraction,
- a device for separating films out of the medium fraction and the coarse fraction is located after each of the two sieves,
- a device for eliminating ferromagnetic materials from the medium fraction and the fine fraction is located after each of the two sieves,
- furthermore, a device for eliminating non-ferrous metals from the medium fraction and the fine fraction is located after each of the two sieves,
- a ballistic sorter for separating off paper and remaining plastics films is located after the non-ferrous metal separator and the magnetic separator for the medium fraction,
- a means for the separate discharge of the plastic pieces on to a conveyor belt is located after the sorter, with the separating means ensuring strict arrangement of the plastic pieces one behind the other on the conveyor belt,
- a means for detecting the size of the separated plastic pieces is located in the immediate vicinity of the conveyor belt,
- a means for the non-contacting performance of a spectral analysis on the separated plastic pieces is arranged in the direction of transportation after the size-detection means,
- a multi-channel discharge device is arranged in the direction of transportation after the means for spectral analysis, each of the plurality of discharge channels of which is allocated to a given plastics material,
- a computer control means is provided which is connected signal-wise to the transport means, the size detection means, the means for spectral analysis and the discharge device and discharges plastic pieces of the same type in each case in the same manner from the conveyor belt in order to form pure fractions of plastics materials.

13. An installation according to Claim 12, characterised in that the means for spectral analysis is a spectrometer for the region of ultraviolet, visible and infrared radiation, which effects the production of the spectrum by means of pulsed laser radiation.

14. An installation according to one of Claims 12 to 13, characterised in that the means for determining the size of the plastic pieces is designed as a light barrier system or as a camera system.

15. An installation according to one of Claims 12 to 14, characterised in that a means for colour recognition of the plastic pieces which is connected signal-wise to the computer control means is arranged before the discharge device.

16. An installation according to one of Claims 12 to 15, characterised in that the mesh width of the fine sieve is 25 mm to 40 mm.

17. An installation according to one of Claims 12 to 16, characterised in that the mesh width of the coarse sieve is restricted to 500 mm, preferably to 400 mm.

18. An installation according to one of Claims 12 to 17, characterised in that the discharge device is designed as a system of compressed-air nozzles arranged in series in the direction of transportation, the direction of blowing of which nozzles is at right-angles to the direction of transportation in each case and which are each associated with separate collecting means.

## Revendications

1. Procédé pour l'extraction commandée par calculateur de fractions en matière synthétique de même espèce, de mélanges constitués en grande partie de parties en matière synthétique, en particulier des matériaux d'emballage, caractérisé par la combinaison des étapes de procédé suivantes :
a) par un filtrage grossier, on extrait au moins les parties de plus de 600 mm du mélange, comme fraction grossière,
b) pour séparer une fraction fine d'une fraction moyenne, le mélange restant de l'étape a) du procédé est soumis à un filtrage fin avec une largeur de mailles d'au moins 15 mm à 50 mm au maximum,
c) les fractions moyenne et grossière sont soumises à un filtrage de feuilles,
d) les fractions moyenne et fine sont soumises à chaque fois à un filtrage magnétique pour séparer des matériaux ferromagnétiques,
e) les fractions moyenne et fine sont soumises à chaque fois à une séparation de métaux non ferreux,
f) la fraction moyenne est soumise, après la mise en oeuvre des étapes a) à e) du procédé, à un triage balistique, par lequel en particulier le papier et des feuilles restantes sont séparés en tant que fraction volante,
g) les parties de la fraction moyenne restante de l'étape f) du procédé sont individualisées et sont transportées de façon stricte les unes derrière les autres à travers les autres étapes du procédé,
h) la taille des parties individuelles de la fraction moyenne individualisée est déterminée à chaque fois par la commande par calculateur par une mesure optoélectronique,
i) le type de matériau de chaque partie individuelle est déterminé lors du transport par une analyse spectrale,
k) le résultat de l'analyse spectrale est transmis à la commande par calculateur, et
l) en correspondance au résultat de l'analyse spectrale, un dispositif d'éclusage est commandé par la commande par calculateur, dispositif d'éclusage qui transporte chaque partie avec d'autres parties de même espèce dans un dispositif de collecte correspondant.

2. Procédé selon la revendication 1,
caractérisé en ce que, pour la mise en oeuvre de l'analyse spectrale, un échantillon de matériau de chaque partie est vaporisé au moyen d'une impulsion à rayonnement laser déclenchée par la commande par calculateur et dirigée sur la partie, et est transformé en un plasma.

3. Procédé selon l'une des revendications 1 ou 2,
caractérisé en ce que la reconnaissance de matériau est effectuée par la comparaison des lignes spectrales à chaque fois déterminées dans le domaine du rayonnement ultraviolet, visible et infrarouge, à des spectres de comparaison stockés dans la commande par calculateur et associés à des types individuels de matières synthétiques.

4. Procédé selon l'une des revendications 1 à 3,
caractérisé en ce que la détermination de matériau pour chaque partie est effectuée par au moins deux, de préférence trois, prélèvements effectués à des endroits différents de la partie.

5. Procédé selon l'une des revendications 1 à 4,
caractérisé en ce qu'en plus de l'analyse spectrale pour la reconnaissance du type de matériau, on effectue une détermination de la couleur pour chaque partie, dont le résultat est transmis à la commande par calculateur pour réaliser un tri supplémentaire des parties en fonction de la couleur.

6. Procédé selon la revendication 5,
caractérisé en ce que la reconnaissance de couleur est effectuée à l'aide d'une caméra à couleur.

7. Procédé selon la revendication 5,
caractérisé en ce que la reconnaissance de couleur est effectuée par l'exploitation de portions caractéristiques du spectre d'ondes détecté par l'analyse spectrale.

8. Procédé selon l'une des revendications 1 à 7,
caractérisé en ce que la détermination de la taille des parties est effectuée au moyen de barrages photoélectriques.

9. Procédé selon l'une des revendications 1 à 7,
caractérisé en ce que la détermination de la taille des parties est effectuée au moyen d'une caméra.

10. Procédé selon l'une des revendications 1 à 9,
caractérisé en ce que le filtrage pour séparer la fraction fine est réalisé avec une largeur de mailles de 25 à 40 mm.

11. Procédé selon l'une des revendications 1 à 10,
caractérisé en ce que par le filtrage grossier, la taille maximale des parties de la fraction moyenne est limitée à 500 mm, de préférence à 400 mm.

12. Installation pour la mise en oeuvre du procédé selon la revendication 1,
caractérisée par la combinaison des caractéristiques suivantes :
- on prévoit au moins un filtre grossier avec une largeur de mailles de 300 à 600 mm et un filtre fin monté en aval avec une largeur de mailles de 15 à 50 mm, pour partager le mélange en une fraction grossière, une fraction moyenne et une fraction fine,
- en aval des deux filtres, est agencé à chaque fois un dispositif pour la séparation de feuilles de la fraction moyenne et de la fraction grossière,
- en aval des deux filtres est agencé à chaque fois un dispositif pour extraire des matériaux ferromagnétiques de la fraction moyenne et de la fraction fine,
- en aval des deux filtres est agencé en outre à chaque fois un dispositif pour extraire des métaux non ferreux de la fraction moyenne et de la fraction fine,
- en aval du séparateur de métaux non ferreux et du séparateur magnétique pour la fraction moyenne est agencé un trieur balistique pour séparer le papier et des feuilles restantes en matière synthétique,
- en aval du trieur est agencé un dispositif pour la transmission individualisée des parties en matière synthétique sur une bande de transport, le dispositif d'individualisation garantissant un agencement strict des parties en matière synthétique les unes derrière les autres sur la bande de transport,
- à proximité de la bande de transport est agencé un dispositif pour déterminer la taille des parties individualisées en matière synthétique,
- dans le sens de transport en aval du dispositif de détermination de la taille, est agencé un dispositif pour la mise en oeuvre sans contact d'une analyse spectrale sur les parties individualisées en matière synthétique,
- dans le sens de transport en aval du dispositif pour l'analyse spectrale, est agencé un dispositif d'éclusage à plusieurs canaux, dont les canaux d'éclusage sont associés à chaque fois à une matière synthétique déterminée, et
- on prévoit une commande par calculateur qui est reliée, d'un point de vue de technique de signal, au dispositif de transport, au dispositif de détermination de la taille, au dispositif pour l'analyse spectrale et au dispositif d'éclusage, et qui retire des parties identiques en matière synthétique à chaque fois de manière identique de la bande de transport pour former des fractions de matières synthétiques de même espèce.

13. Installation selon la revendication 12,
caractérisée en ce que le dispositif pour l'analyse spectrale est un spectromètre pour le domaine du rayonnement ultraviolet, visible et infrarouge, qui effectue la génération du spectre au moyen d'un rayonnement laser en régime pulsé.

14. Installation selon l'une des revendications 12 ou 13, caractérisée en ce que le dispositif pour la détermination de la taille des parties en matière synthétique est réalisé sous forme d'un système à barrage photoélectrique ou d'un système à caméra.

15. Installation selon l'une des revendications 12 à 14, caractérisée en ce qu'en amont du dispositif d'éclusage est agencé un dispositif relié, d'un point de vue de technique de signal, à la commande par calculateur, pour reconnaître la couleur des parties en matière synthétique.

16. Installation selon l'une des revendications 12 à 15, caractérisée en ce que la largeur de mailles du filtre fin vaut 25 à 40 mm.

17. Installation selon l'une des revendications 12 à 16, caractérisée en ce que la largeur de mailles du filtre grossier est limitée à 500 mm, de préférence à 400 mm.

18. Installation selon l'une des revendications 12 à 17, caractérisée en ce que le dispositif d'éclusage est réalisé sous forme d'un système à buses à air comprimé, agencées les unes derrière les autres en direction de transport, dont la direction de soufflage est à chaque fois transversale à la direction de transport, et qui sont associées à chaque fois à des dispositifs de collecte séparés.
